# EUROPEAN PATENT APPLICATION

(11) **EP 1 754 474 A1**
(43) Date of publication of application: **21.02.2007**
(21) Application number: 05002126.0
(22) Date of filing: 02.02.2005
(51) Int. Cl.: A61K 31/137, A61P 25/28

(54) **Use of S-Clenbuterol**

(71) Applicant: EUCRO European Contract Research GmbH & Co. KG, 41460 Neuss (DE)
(72) Inventor: Greb, Wolfgang Dr., 40489 Düsseldorf (DE); Krieglstein, Josef, 35091 Cölbe (DE); Culmsee, Carsten Dr., Butenandstrasse 5-13, 81377 München (DE)
(74) Representative: Christophersen, Ruth

(57) **Abstract**

The use of S-Clenbuterol for restoring and/or maintaining the function of partially or completely damaged/degenerated cells in the central nervous system and/or other nerve cells is claimed. The use of S-Clenbuterol leads to activation of astrocytes and initiation of endogenous processes of neuroprotection, it thus being possible for the damage or destruction of nerve cells to be reduced and, in some cases, even prevented.

## Description

The present invention relates to the use of S-Clenbuterol for the treatment of neurodegenerative diseases.

The human brain is a highly complicated organ with more than 100 billion nerve cells (= neurons) and about 10 000 connections (= synapses) per cell. The brain is the central organ of the conscious and unconscious processing of the stimuli acting on the human body, of thought and feeling, of deliberate action, of learning and of memory. One of the most important functions of the human brain is information processing in speech; also control center for a large number of organ functions, and of breathing, of the heart rate and of temperature regulation.

A large number of diseases lead to the damage and even death of nerve cells and/or to a reduction in synapses and thus to a restriction of brain function. Examples of such pathological states are Alzheimer's disease, cerebrovascular dementias, Parkinson's disease, Pick's disease, Huntington's chorea, amyotrophic lateral sclerosis, Lewy body dementia, stroke and brain trauma such as cerebral contusion and concussion, and injuries to the brain and spinal cord or transverse lesions, spina bifida, and diseases of the inner ear, for example diseases associated with the occurrence of tinnitus, such as subacute or chronic tinitus, sudden loss of hearing, Menière's disease, and diseases associated with a restriction of audition or with the reduction in vision etc. There is as yet no clinically established neuroprotective therapy of said pathological states. Only symptoms, but not the causes thereof, are treated.

The aim of causal therapy is to prevent the death of nerve cells.

There is at present no established therapy with which it is possible for nerve cells to be protected from damage or regenerated. An essential element of the therapy applied at present to the abovementioned disorders is to prevent indirect or secondary damage to nerve cells, such as, for example, to increase cerebral blood flow or restore blood flow if a vessel was occluded. However, this type of therapy is successful only, if at all, when it can be employed rapidly after the acute event.

Junker et al Eur J Pharmacol 446(2002) p.25-36 have described positive effects of racemic Clenbuterol in preclinical models of neuroprotection. The mechanism of such action seems to be mediated via β-receptors. In the US-Patent 3,536,712 racemic mixtures of the compounds of the general formula are disclosed 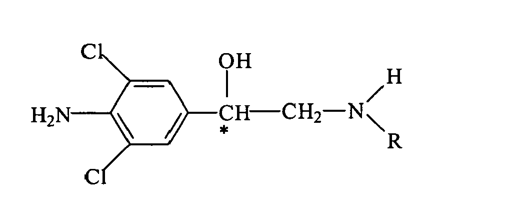 wherein R is a branched alkyl residue with 3 or 4 carbon atoms, and acid addition salts thereof. The compound wherein R is the t-butyl residue, is also known under the INN Clenbuterol. The disclosed compounds are used as pharmaceuticals, in particular for the treatment of asthma.

DE 22 12 600 discloses that the optical active antipodes of the compounds disclosed in the US-Patent 3,536,712 have an inhibiting effect on adrenergic β-receptors. According to the disclosure the d-S-compounds of the formula I have a selective effect on the β₁-receptors and the 1-(-)-compounds of the general formula I have a preferred effect on the β₂-receptors.
Biological activity for both isomers of Clenbuterol has so far been shown only for its anabolic properties. In CHIRALITY 12: 637-648 (2000) von Deutsch et al. tested the anabolic effects on the skeletal muscles and protein synthesis. There were no remarkable differences in the effects for both enantiomers on the skeletal muscles. None of the enantiomers had any measurable effect on bone mass. It seems likely that the anabolic effects are mediated by a different mechanism.

However, the remarks made in German Patent 22 12 600 concerning the β-receptor mediated activities of each optical active enantiomer were proven wrong by subsequent investigations. According to Acta pharmacol. at toxicol. 1985, 56, 221-227 Waldeck and Widmark found that of β-adrenoceptor agonists or antagonists the (-)-isomer is the pharmacologically active form, whereas the optical antipode is more or less not active. In contrast to DE 2212 600 they found no selective B 1-antagonistic activity. The results of German Patent 22 12 600 were not proven.
In WO 9916 430 is mentioned that for ß2-agonists like salbutamol, salmeterol etc, the R-isomers carry the β-agonist activity, whereas the S-isomers do not have affinity to the receptor.

Bramuglia and Robio tested the relaxant response in rat uterus on Clenbuterol isomers and found that only the (-)-isomer shows the effect and that the S-isomer is not involved in the effects (pharmacological research: the official journal of the Italian pharmacological society; Vol: 43(2); p. 151-4/200102).

Also Martin at al. found according to European Journal of Pharmacology, 117 (1985) 127-129 that the activity in different psychopharmacological tests was based only on the (-)-enantiomer whereas S-Clenbuterol did not show any CNS-effect.

The cited documents show that scientists, skilled in the art, are of the opinion that from the Clenbuterol isomers only the (-)-enantiomer (R-configuration) shows pharmacological activity.

Surprisingly it was found now that in administration of Clenbuterol or its isomers in neurodegenerative diseases only the S-enantiomer shows any activity.

Subject of the present invention is therefore the use of S-Clenbuterol for the preparation of a medicament for restoring and/or maintaining the function of partially of completely damaged cells of the central nerves systems and/or other nerve cells.

S-Clenbuterol used according to the present invention shows also in acute status, such as stroke, immediately a positive effect on the restoration of nerve cells and subsequently an accelerated convalescence of the at least partial palsied patients.

For the purposes of the present invention, "damaged cell" means that the cell has been damaged by external effects or internal effects in the body or the cell is partially or completely destroyed in the sense of degeneration through processes taking place in the cell, which may be associated with an impairment of body functions. The term "cell damage" includes both damage to individual cells or cell types and damage to strings or tracts of nerve cells.

The nerve cells include, besides the cells of the central nervous system, also the cells of the spinal cord and all other nerve cells present in the body.

S-Clenbuterol used according to present invention may be administered in the amounts customary for this pharmaceutical, in particular in an amount of from 0.01 to 100 mg/day, preferably in an amount of from 0.01 to 5 mg/day.

S-Clenbuterol can be used in its pure form or as a physiologically acceptable acid addition salt. Preferred acid addition salts are hydrogen chloride, hydrogen bromide, sulfate, perchlorate, sulfonate, toloylsulfonate, etc., preferably the hydrogen chloride is used, especially the ammonia or tetramethyl ammonia hydrogen chloride.

In a further possible embodiment of the present invention, the β-adrenergic agonists are employed in combination with NMDA antagonists, thus applying a supplementary or further principle of action.

NMDA antagonists, such as, for example, the adamantane derivatives, are known compounds which are frequently also employed for the treatment of CNS diseases. Thus, for example, the dopaminergic effect of amantadine (1-adamantanamine) is known.

European patent application EP 392 059 describes the use of adamantane derivatives for the prevention and treatment of cerebral ischemia. According to this publication, the destruction of brain cells following an ischemia is protectively prevented through the use of the adamantane derivatives in that the adamantane derivatives are employed as antagonists for the NMDA receptor channels of the nerve cells in the brain.

Adamantane derivatives having formula I are preferably employed 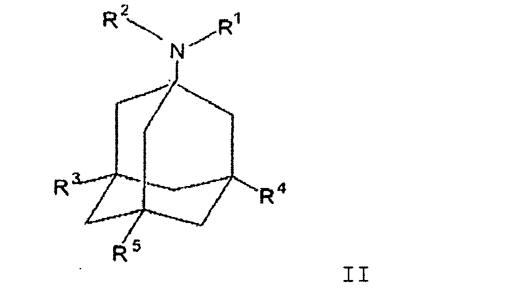 in which R¹ and R² are identical or different and are hydrogen or a straight-chain or branched C₁-C₆₋alkyl group, or together with the N atom may represent a heterocyclic group having 5 or 6 ring atoms,
R³ and R⁴ are identical or different and are hydrogen, a straight-chain or branched C₁-C₆-alkyl group or a C₅-C₆-cycloalkyl group or a vinyl group, and
R⁵ is hydrogen or a straight-chain or branched C₁₋C₆-alkyl group.

The adamantane derivatives having formula II can be employed in the form of their compounds described by formula II or in the form of their pharmaceutically acceptable salts. Pharmaceutically acceptable salts which can preferably be employed include the acid addition salts such as the hydrochlorides, hydrobromides, sulfates, acetates, succinates, tartrates, with preference for the hydrochlorides.

Preferred compounds having formula II are those in which R¹, R² and R⁴ are hydrogen and R³ and R⁵ are a methyl and/or ethyl group.

In a particularly preferred compound, R¹, R² and R⁴ are hydrogen and R³ and R⁵ are a methyl radical, or the hydrochloride thereof. This compound is known under the INN memantine.

The S-Clenbuterol used according to the invention and, where appropriate, further customary drug substances which do not adversely affect the therapy, or which support it, and conventional ingredients, can be present in pharmaceutically customary dosage forms, in particular as solution, suspension, emulsion, tablets, suppository, etc. Use in special formulations such as liposomes, nanosomes, slow-release pellets etc. is also possible. They can be administered in a conventional way, for example orally, parenterally, intravenously, by inhalation, nasally, rectally, intraventricularly, into the ear or eye, dermally, intra-arterially, intraperitoneally and/or intramuscularly or as an implant. The mode of administration is preferably selected so that the impaired cells can be reached by the drug substance of the invention in the fastest possible manner.

The S-Clenbuterol used according to the invention are particularly suitable for producing medicaments for the treatment of neurodegenerative diseases. Examples of such diseases are, but not limited to, Alzheimer's disease, cerebrovascular dementias, Parkinson's disease, Pick's disease, Huntington's chorea, amyotrophic lateral sclerosis, multiple sclerosis, Lewy body dementia, stroke and brain trauma such as cerebral contusion and concussion, and injuries to the brain and spinal cord or transverse lesions, spina bifida, and diseases of the inner ear, for example diseases associated with the occurrence of tinnitus, such as subacute or chronic tinitus, sudden loss of hearing, Menière's disease, and diseases associated with a restriction of audition or with the reduction in vision etc.

In a further embodiment of the present invention, S-Clenbuterol is employed to restore and/or maintain the function of cells of the central nervous system which have been partially or completely damaged by encephalopathy and/or of other nerve cells. Encephalopathy is one of the pathological non-inflammatory changes in the brain with variable neurological and/or mental symptoms. Examples of encephalopathies which can be treated according to the invention with S-Clenbuterol are, but not limited to, toxic encephalopathy, diabetic encephalopathy, hepatic encephalopathy, hypertensive encephalopathy, metabolic encephalopathy, such as encephalopathy caused by metabolic disturbances, e.g. associated with enzymopathies, endogenous disturbances, renal failure (uremic encephalopathy), liver diseases, disturbances of the water/electrolyte or acid/base balance, myoclonic infantile encephalopathy (Kinsboorne syndrome), infantile postictereca encephalopathy (bilirubin encephalopathy), postcombustional encephalopathy, encephalopathy caused by heavy metals, in particular by inorganic and organic heavy metal compounds such as, but not limited to, compounds of lead, mercury, and amalgam, thallium, bismuth, aluminum, nickel and any mixtures of these compounds and their metal alloys, toxic encephalopathy caused by alcohol, bovine spongiform encephalopathy (BSE), supcortical progressive encephalopathy, traumatic encephalopathy.

In a further embodiment of the present invention, S-Clenbuterol used according to the invention is employed to prevent the aforementioned diseases.

In a further embodiment of the present invention, the S-Clenbuterol compound used according to the invention is employed as additive(s) for culture media to promote growth and/or differentiation and/or protection of mammalian cells and human cells.

## Claims

1. The use of S-Clenbuterol for the preparation of a medicament for restoring and/or maintaining the function of partially or completely damaged cells of the central nervous system and/or other nerve cells.

2. The use as claimed in claim 1, **characterized in that** S-Clenbuterol is administed in an amount of from 0.01 to 100 mg/day, in particular from 0,01 to 5 mg a day.

3. The use as claimed in any of claims 1 or 2, **characterized in that** NMDA antagonists are used.

4. The use as claimed in any of claims 1 to 3, **characterized in that** the neurodegenerative diseases are selected from Alzheimer's disease, cerebrovascular dementias, Parkinson's disease, Pick's disease, Huntington's chorea, amyotrophic lateral sclerosis, Lewy body dementia, stroke and/or brain trauma such as cerebral contusion and concussion, and injuries to the brain and spinal cord or transverse lesions, spina bifida, and diseases of the inner ear, for example diseases associated with the occurrence of tinnitus, such as subacute or chronic tinitus, sudden loss of hearing, Meniere's disease, and diseases associated with a restriction of audition or with the reduction in vision etc.

5. The use as claimed in any of claims 1 to 3, **characterized in that** the neurodegenerative diseases are selected from toxic encephalopathy, diabetic encephalopathy, hepatic encephalopathy, hypertensive encephalopathy, metabolic encephalopathy, such as encephalopathy caused by metabolic disturbances, e.g. associated with enzymopathies, endogenous disturbances, renal failure (uremic encephalopathy), liver diseases, disturbances of the water/electrolyte or acid/base balance, myoclonic infantile encephalopathy (Kinsboorne syndrome), infantile postictereca encephalopathy (bilirubin encephalopathy), postcombustional encephalopathy, encephalopathy caused by heavy metals, in particular by inorganic and organic heavy metal compounds such as compounds of lead, mercury, and amalgam, thallium, bismuth, aluminum, nickel and any mixtures of these compounds and the metal alloys, toxic encephalopathy caused by alcohol, bovine spongiform encephalopathy (BSE), sup cortical progressive encephalopathy, traumatic encephalopathy.

6. The use as claimed in any of claims 1 to 3, **characterized in that** the compounds are employed for preventing neurodegenerative diseases.

7. The use as claimed in any of claims 1 to 3 as additive for culture media to promote growth and/or differentiation and/or protection of mammalian cells and human cells.
